# EUROPEAN PATENT APPLICATION

(11) **EP 3 527 159 A2**
(43) Date of publication of application: **21.08.2019**
(21) Application number: 19157516.6
(22) Date of filing: 15.02.2019
(51) Int. Cl.: A61B 34/20

(54) **3D RECONSTRUCTION AND GUIDANCE BASED ON COMBINED ENDOBRONCHIAL ULTRASOUND AND MAGNETIC TRACKING**

(30) Priority: 15.02.2018 US 201862631250 P; 07.02.2019 US 201916269821
(71) Applicant: Covidien LP, Mansfield, MA 02048 (US)
(72) Inventor: KOPEL, Evgeni, 4482000 Barkan (IL); BIRENBAUM, Ariel, 4672559 Herzliya (IL); KRIMSKY, William S., Forest Hill, Maryland 21050 (US)
(74) Representative: Maschio, Antonio

(57) **Abstract**

Disclosed are systems and methods for performing surgical procedures. An illustrative surgical system includes an electromagnetic (EM) tracking system including a EM field generator, a surgical tool, a display device, and a computing device configured to receive image data of the surgical site, identify structures in the image data, identify a target in the image data, generate a three-dimensional (3D) model of the surgical site, determine a pathway to the target, display the pathway to the target, determine a position of the surgical tool within the surgical site, register the 3D model to the surgical site, receive ultrasound image data from an ultrasound sensor, and generate a 3D volume rendering of the target.

## Description

### CROSS REFERENCE TO RELATED APPLICATION

This application claims the benefit of the filing date of provisional U.S. Patent Application No. 62/631,250, filed February 15, 2018, the entire contents of which are incorporated herein by reference.

### INTRODUCTION

The present disclosure relates to the generation of visual guidance for surgical procedures and, more particularly, to systems and methods for capturing ultrasound images of structures within a patient's chest and generating three-dimensional renderings of the ultrasound images to provide visual guidance during the surgical procedures..

### BACKGROUND

Minimally-invasive surgical procedures have become a common and effective means for diagnosing and treating a variety of medical conditions. Endobronchial navigation is one type of such minimally-invasive surgical procedure, and involves insertion of one or more surgical instruments via a bronchoscope and/or other catheter guide assembly into a patient's airways, and navigating the catheter through the airway tree and/or parenchyma to a diagnosis or treatment site. Various systems and surgical instruments have been developed to aid clinicians during such endobronchial navigation procedures, such as to assist with placing a catheter or other surgical instrument at a desired diagnosis or treatment site. However, existing systems rely on optical images provided by cameras in the bronchoscope or catheter and/or computed tomography (CT) images acquired pre-procedure. Optical images alone are often insufficient for accurately guiding surgical instruments to a desired diagnosis or treatment site because optical cameras cannot capture images of structures behind airway walls or behind obstructions in the airways. Additionally, due to the complex structure of a patient's bronchial (airway) tree, it is often difficult to identify exactly where in the airway tree the bronchoscope or catheter is located. As such, the systems and methods described hereinbelow provide improvements in imaging and visualization techniques for use while navigating within a patient's airways during surgical procedures.

### SUMMARY

Provided in accordance with the present disclosure are systems and methods for performing surgical procedures. In an aspect of the present disclosure, an illustrative surgical system includes an electromagnetic (EM) tracking system including a EM field generator configured to generate an EM field about a surgical site, a surgical tool including an ultrasound sensor and an EM sensor, a display device, and a computing device including a processor and a memory storing instructions which, when executed by the processor, cause the computing device to receive first image data of the surgical site, identify a structure in the first image data, identify a target in the first image data, generate a three-dimensional (3D) model of the surgical site based on the first image data and the identified structure and target, determine a pathway to the target, cause the display device to display the pathway to the target, determine a position of the surgical tool within the surgical site based on tracking data received from the EM tracking system, the tracking data indicating a position of the EM sensor within the EM field, register the 3D model to the surgical site, receive second image data from the ultrasound sensor, generate a 3D volume rendering of the target, and cause the display device to display the 3D volume rendering.

In another aspect, the surgical tool further includes an expandable balloon.

In yet another aspect, the instructions, when executed by the processor, further cause the computing device to cause the display device to display guidance for positioning the surgical tool relative to the target.

In still another aspect, the instructions, when executed by the processor, further cause the computing device to cause the display device to display guidance for moving the surgical tool relative to the target while the ultrasound sensor captures the second image data.

In another aspect, the instructions, when executed by the processor, further cause the computing device to cause the display device to display a view of the 3D model showing regions of the surgical site for which second image data has been received with a first characteristic, and regions of the surgical site for which second image data has not been received with a second characteristic.

In yet another aspect, the instructions, when executed by the processor, further cause the computing device to cause the display device to display a view of the first image data showing regions of the surgical site for which second image data has been received with a first characteristic, and regions of the surgical site for which second image data has not been received with a second characteristic.

In still another aspect, the instructions, when executed by the processor, further cause the computing device to process the second image data to remove artifacts from the second image data

In yet another aspect, the 3D volume rendering is generated based on at least a portion of the second image data and the determined position of the surgical tool.

In still another aspect, the instructions, when executed by the processor, further cause the computing device to register the 3D volume rendering to the 3D model.

In yet another aspect, the instructions, when executed by the processor, further cause the computing device to register the 3D volume rendering to the first image data.

In still another aspect, the instructions, when executed by the processor, further cause the computing device to update the 3D volume rendering based on the identified structure.

In yet another aspect, the structure includes one or more of a bronchial tree, a vascular tree, a lymphatic tree, a lesion, a cyst, an esophagus, a pleural surface, a lymph node, an organ, and a marker.

In still another aspect, the instructions, when executed by the processor, further cause the computing device to cause the display device to display the updated 3D volume rendering.

In yet another aspect, the instructions, when executed by the processor, further cause the computing device to cause the display device to display an individual slice image of the 3D volume rendering.

In still another aspect, the instructions, when executed by the processor, further cause the computing device to update the registration of the 3D model to the surgical site based on the registration of the 3D volume rendering to the 3D model.

In yet another aspect, the instructions, when executed by the processor, further cause the computing device to update the 3D model based on the second image data.

In another aspect of the present disclosure, an illustrative surgical system includes an EM tracking system including a EM field generator configured to generate an EM field about a surgical site, a surgical tool including an ultrasound sensor and an EM sensor, and a computing device including a processor and a memory storing instructions which, when executed by the processor, cause the computing device to receive first image data of the surgical site, generate a 3D model of the surgical site based on an identified target in the image data, determine a pathway to the target, determine a position of the surgical tool within the surgical site based on a determined position of the EM sensor within the EM field, receive second image data from the ultrasound sensor, and generate a 3D volume rendering of the target based on the second image data.

In another aspect, the instructions, when executed by the processor, further cause the computing device to register the 3D volume rendering of the target to the 3D model of the surgical site.

In another aspect of the present disclosure, an illustrative method for generating visual guidance for a surgical procedure includes receiving first image data of a surgical site, generating a three-dimensional (3D) model of the surgical site, determining a pathway to a target identified in the first image data, displaying the pathway to the target on a display, determining a position of an electromagnetic (EM) sensor within an EM field generated about the surgical site, receiving second image data of the target from an ultrasound sensor, and generating a 3D volume rendering of the target based on the second image data.

In another aspect, the method includes registering the 3D volume rendering of the target to the 3D model of the surgical site.

Any of the above aspects and embodiments of the present disclosure may be combined without departing from the scope of the present disclosure.

### BRIEF DESCRIPTION OF THE DRAWINGS

Various aspects and features of the present disclosure are described hereinbelow with references to the drawings, wherein:
FIG. 1 is a schematic diagram of a system for planning and performing treatment of an area of a patient's chest, according to an embodiment of the present disclosure;
FIG. 2 is a block diagram of a computing device forming part of the system of FIG. 1;
FIGS. 3A, 3B, 3C, and 3D show a flowchart of an illustrative method for predicting spread of disease based on a lymphatic tree map, according to an embodiment of the present disclosure;
FIG. 4 is a view of an illustrative graphical user interface showing a 3D model of at least a portion of the patient's chest which may be displayed by the computing device of FIG. 3 during performance of the method of FIGS. 3A-3D, according to an embodiment of the present disclosure;
FIG. 5 is a view of another illustrative graphical user interface showing a 3D model of at least a portion of the patient's chest which may be displayed by the computing device of FIG. 2 during performance of the method of FIGS. 3A-3D, according to an embodiment of the present disclosure;
FIG. 6 is a view of yet another illustrative graphical user interface showing a 3D model of at least a portion of the patient's chest which may be displayed by the computing device of FIG. 2 during performance of the method of FIGS. 3A-3D, according to an embodiment of the present disclosure;
FIG. 7 is another view of the graphical user interface of FIG. 6 showing additional details that may be displayed by the computing device of FIG. 2 during performance of the method of FIGS. 3A-3D, according to an embodiment of the present disclosure;
FIG. 8 is yet another view of the graphical user interface of FIGS. 6 and 7 showing additional details that may be displayed by the computing device of FIG. 2 during performance of the method of FIGS. 3A-3D, according to an embodiment of the present disclosure; and
FIG. 9 is a view of a graphical user interface showing a summary of treatment procedures performed during the performance of the method of FIG. 3, according to an embodiment of the present disclosure.

### DETAILED DESCRIPTION

The present disclosure relates to systems and methods for providing visual guidance during surgical procedures. More particularly, the disclosure relates to capturing ultrasound image data of structures inside a patient's chest, creating three-dimensional (3D) renderings of the structures based on the ultrasound image data, registering the 3D renderings to a 3D model of the patient's chest generated based on pre-procedure radiographic image data of the patient's chest, and displaying the 3D renderings in conjunction with and/or in addition to the 3D model during navigation of surgical tools about the patient's chest.

Pre-procedural imaging of the patient's chest may be performed to create a visual representation, such as a three-dimensional (3D) model of a patient's chest, including lumens such as the bronchial, vascular, and lymphatic trees, pleural surfaces and fissures of the patient's lungs, and/or tumors or other aberrant structures that may be present in the patient's lungs. The 3D model may be generated using one or more software applications executing on a computer. The application may, for example, generate the 3D model or map of the patient's chest based on radiographically obtained images, such as computed tomography (CT) images, magnetic resonance imaging (MRI) images, positron emission tomography (PET) images, X-ray images, cone-beam computed tomography (CBCT) images, and/or any other applicable imaging modality. The images may be processed to create a volume of image data of the patient's chest based upon which the 3D model is generated. The image data and/or 3D model may further be processed to identify one or more targets, such as tumors, lesions, or other aberrant structures, in the patient's chest. For example, the application may identify the locations of lumens, such as airways, blood vessels, and/or lymphatic structures from the radiographic image data, and further determine the locations of one or more diagnostic or treatment targets (referred to hereinafter as "targets").

In some embodiments, the application may then receive or load a model lymph node map, such as the International Association for the Study of Lung Cancer (IASLC) map, which includes the locations of lymph nodes in a model patient's body. Thereafter, the application may fit the model lymph node map to the 3D model to align the model map with the real patient's body and the identified structures in the patient's chest to identify and label lymph nodes and/or other structures on the 3D model. Additionally, as further described in U.S. Provisional Patent Appl. No. 62/624,905, entitled MAPPING DISEASE SPREAD, filed on February 1, 2018, by William S. Krimsky, the entire contents of which are incorporated herein by reference, one or more lymphatic tree maps of the patient's lymphatic system may be generated based on the model lymph node map fitted to the 3D model. The generated lymphatic tree maps may further be fitted and/or updated based on known locations of lymph nodes in the patient's chest.

The 3D model, radiographic image data, and/or lymphatic tree map may then be displayed to and viewed by a clinician and/or surgeon to plan a medical procedure, such as a diagnostic or treatment procedure, including biopsy, ablation, radiation, and/or surgical or interventional procedure. For example, the clinician may review the 3D model, radiographic image data, and/or lymphatic tree map to identify one or more structures, such as lymph nodes, lesions, and/or other targets for diagnosis and/or sampling (such as biopsy). The application may then determine a path to the identified structures to assist a clinician with navigating one or more surgical tools through the patient's airways to the structures, as further described below.

At the start of a navigation procedure, the 3D model is registered to the patient's body, as further described below. One or more surgical tools are then tracked via an electromagnetic tracking system as the tools are navigated via the patient's airways to one of the structures. Once a surgical tool is navigated to one of the structures, ultrasound image data of the structure may be captured via an ultrasound sensor coupled to or included in the surgical tool. A 3D rendering of the structure may then be generated based on the ultrasound image data. The 3D rendering is then registered to the 3D model, radiographic image data, and/or lymphatic tree map based on the known position of the surgical tool relative to the 3D model when the ultrasound image data were obtained. The 3D rendering and/or the 3D model, as well as the registration of the 3D model to the patient's body, may then be updated and/or augmented. The 3D rendering, the 3D model, and/or a fusion of the two may then be displayed during a subsequent diagnostic or treatment procedure.

The systems and methods described herein are useful in various planning and/or navigation contexts for diagnostic and/or treatment procedures performed in the patient's chest. For example, in an embodiment in which a clinician is performing diagnosis of targets in an area of the patient's lungs, the systems and methods may provide the clinician with various views of the patient's lungs and the bronchial, vascular, and lymphatic trees therein. Additionally, as will be described in further detail below, the systems and methods may provide the clinician with the ability to view and/or determine various characteristics of the targets, as well as view the position of surgical tools relative to the targets with greater detail than is possible with conventional systems. These and other aspects of the present disclosure are detailed hereinbelow.

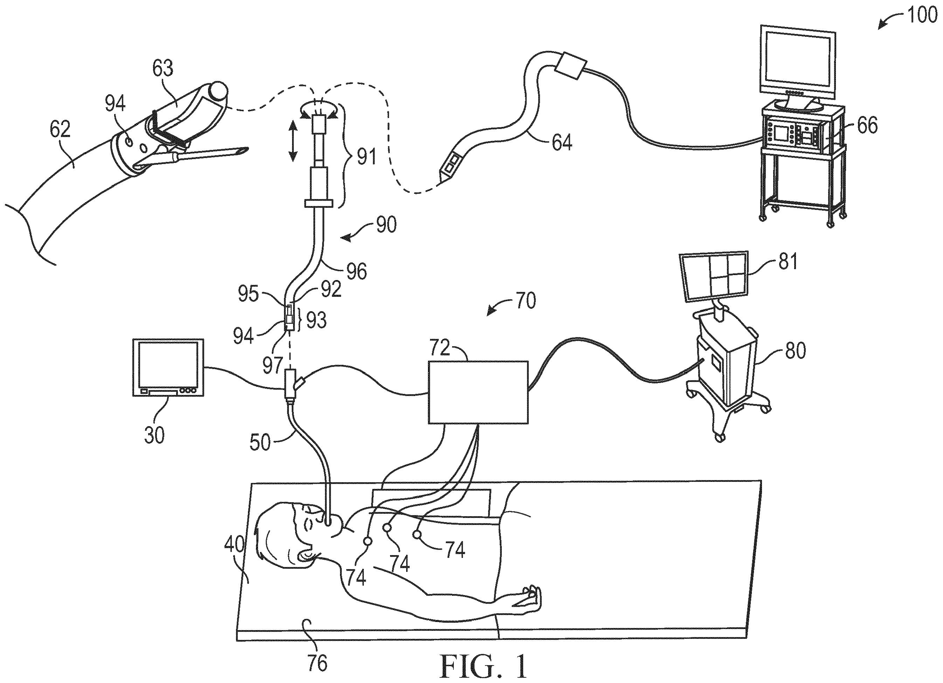

With reference to FIG. 1, an electromagnetic navigation (EMN) system 100 suitable for implementing methods for performing endobronchial diagnostic and/or treatment procedures in an area of a patient's chest is provided in accordance with the present disclosure. One such EMN system 100 is the ELECTROMAGNETIC NAVIGATION BRONCHOSCOPY (ENB) system currently sold by Covidien LP, a division of Medtronic PLC. As shown in FIG. 1, the EMN system 100 is used to perform one or more treatment procedures on a patient supported on an operating table 40. In this regard, the EMN system 100 generally includes a bronchoscope 50, monitoring equipment 30, an electromagnetic (EM) tracking system 70, and a computing device 80.

The bronchoscope 50 is configured for insertion through the patient's mouth and/or nose into the patient's airways. The bronchoscope 50 includes a source of illumination and a video imaging system (not explicitly shown) including at least one optical sensor (such as a camera) which is in operative communication with the monitoring equipment 30, for example, a video display, for displaying the video images received from the video imaging system of the bronchoscope 50. In some embodiments, the bronchoscope 50 further includes an ultrasound sensor (not shown in FIG. 1). The bronchoscope 50 may operate in conjunction with a catheter guide assembly 90. The catheter guide assembly 90 includes an extended working channel (EWC) 96 configured for insertion through a working channel of the bronchoscope 50 into the patient's airways (although the catheter guide assembly 90 may alternatively be used without the bronchoscope 50). The catheter guide assembly 90 further includes a handle 91 connected to the EWC 96, and which can be manipulated by rotation and compression to steer the EWC 96. In the operation of catheter guide assembly 90, a locatable guide (LG) 92, including an EM sensor 94, is inserted into the EWC 96 and locked into position such that the EM sensor 94 extends a desired distance beyond a distal tip 93 of the EWC 96. The location of the EM sensor 94, and thus the distal tip 93 of the EWC 96, within an EM field generated by the EM field generator 76, can be derived by a tracking module 72 and the computing device 80. For a more detailed description of the catheter guide assembly 90, reference is made to commonly-owned U.S. Patent No. 9,247,992, entitled "MICROWAVE ABLATION CATHETER AND METHOD OF UTILIZING THE SAME", filed on March 15, 2013, by Ladtkow et al., the entire contents of which are hereby incorporated by reference.

A six degrees-of-freedom EM tracking system 70, e.g., similar to those disclosed in U.S. Patent No. 6,188,355, entitled "WIRELESS SIX-DEGREE-OF-FREEDOM LOCATOR", filed on December 12, 1997, by Pinhas Gilboa, U.S. Patent No. 6,833,814, entitled "INTRABODY NAVIGATION SYSTEM FOR MEDICAL APPLICATIONS", filed on August 2, 1998, by Gilboa et al., and PCT Publication No. WO/2001/067035, entitled "OBJECT TRACKING USING A SINGLE SENSOR OR A PAIR OF SENSORS", filed on March 9, 2000, by Pinhas Gilboa, the entire contents of each of which are incorporated herein by reference, or any other suitable positioning measuring system, is utilized for performing navigation, although other configurations are also contemplated.

The EM tracking system 70 may be configured for use with the catheter guide assembly 90 to track a position of the EM sensor 94 as it moves in conjunction with the EWC 96 through the airways of the patient, as detailed below. In an embodiment, the EM tracking system 70 includes the tracking module 72, a plurality of reference sensors 74, and an EM field generator 76. As shown in FIG. 1, the EM field generator 76 is positioned beneath the patient. The EM field generator 76 and the plurality of reference sensors 74 are interconnected with the tracking module 72, which derives the location of each reference sensor 74 in the six degrees of freedom. One or more of the reference sensors 74 are placed on or attached to the chest of the patient. The six degrees of freedom coordinates of the reference sensors 74 are sent as data to the computing device 80, which includes an application 81, where the data from the reference sensors 74 are used to calculate a patient coordinate frame of reference.

Although the EM sensor 94 is described above as being included in the LG 92, it is also envisioned that the EM sensor 94 may be embedded or incorporated within a treatment tool, such as a endobronchial ultrasound (EBUS) 62 tool and/or an ablation tool 64, or a diagnostic tool, such as a camera tool, a light sensor, a linear ultrasound tool, etc., where the treatment tool may alternatively be utilized for navigation without need of the LG 92 or the necessary tool exchanges that use of the LG 92 requires. The EM sensor 94 may also be embedded or incorporated within the EWC 96, such as at a distal portion of the EWC 96, thereby enabling tracking of the distal portion of the EWC 96 without the need for a separate LG 92. According to an embodiment, treatment tools 62, 64 are configured to be insertable into the catheter guide assembly 90 following navigation to a target and removal of the LG 92. The EBUS 62 includes at least one ultrasound sensor 63 configured to capture ultrasound images. The ultrasound sensor may be configured to capture ultrasound image data using various frequencies and/or modes of operation, as will be known to those skilled in the art. One example mode of operation includes Doppler. In some embodiments, the EBUS 62 may further include a biopsy tool, such as a needle and/or a brush, which may be used to collect one or more tissue samples from the target. The EBUS 62 may further include one or more expandable balloons which may be used to lock the position of the EBUS 62 during ultrasound imaging and/or while a biopsy procedure is being performed. In embodiments, the EBUS 62 is further configured for use in conjunction with the tracking system 70 to facilitate navigation of the EBUS 62 to the target by tracking the position of EM sensor 94, and thus the EBUS 62, as it is navigated through the patient's airways and manipulated relative to the target. The EBUS 62 may additionally be coupled to an ultrasound workstation (not shown in FIG. 1) and/or the computing device 80 to facilitate capture, processing, and analysis of ultrasound images acquired by the ultrasound sensor 63. The ablation tool 64 is configured to be operated with a generator 66, such as a radio frequency generator or a microwave generator, and may include any of a variety of ablation tools and/or catheters, examples of which are more fully described in commonly-owned U.S. Patent No. 9,259,269, entitled "MICROWAVE ABLATION CATHETER AND METHOD OF USING THE SAME", filed on March 15, 2013, by Ladtkow et al., the entire contents of which are incorporated herein by reference. In addition to the tools described above and/or in the incorporated documents, those skilled in the art will recognize that other tools, including for example RF ablation tools, brachytherapy tools, and others may be similarly deployed and tracked without departing from the scope of the present disclosure.

The computing device 80 includes hardware and/or software, such as an application 81, used to facilitate the various phases of an EMN procedure, as described further below. For example, computing device 80 utilizes radiographic image data acquired from a CT scan, cone beam computed tomography (CBCT) scan, magnetic resonance imaging (MRI) scan, positron emission tomography (PET) scan, X-ray scan, and/or any other suitable imaging modality to generate and display a 3D model of the patient's airways, identify a target on the radiographic image data and/or 3D model (automatically, semi-automatically or manually), and allow for the determination and selection of a pathway through the patient's airways to the target. The 3D model may be presented on a display device associated with the computing device 80, or in any other suitable fashion. An example of the planning software described herein can be found in commonly-assigned U.S. Patent No. 9,459,770, filed by Baker et al. on March 15, 2013, and entitled "PATHWAY PLANNING SYSTEM AND METHOD", the entire contents of which are incorporated herein by reference. Further examples of the planning software can be found in commonly-assigned U.S. Patent No. 9,770,216, entitled "SYSTEM AND METHOD FOR NAVIGATING WITHIN THE LUNG", filed on June 29, 2015, by Brown et al., the entire contents of which are incorporated herein by reference.

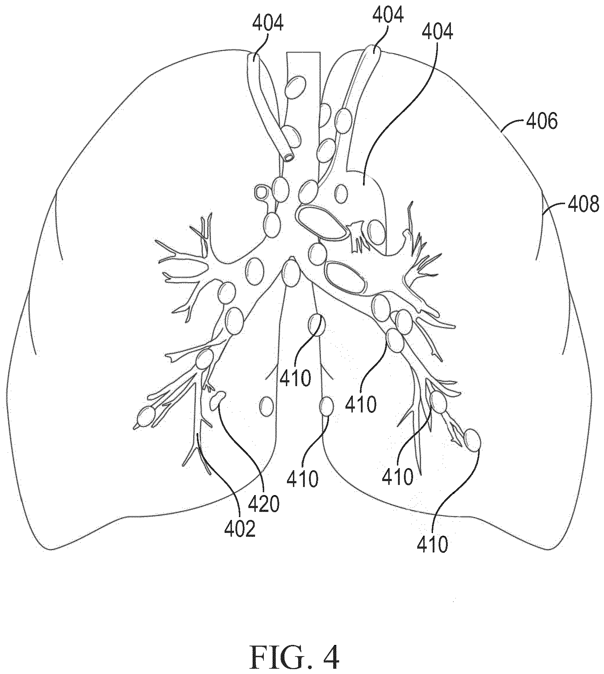

Using the computing device 80, various views of the 3D model may be displayed to and manipulated by a clinician to facilitate identification of a target. As noted above, the target may be one or more lesions or lymph nodes, a surgical site where treatment is to be performed, and/or a portion of, entire lobe, or multiple lobes of the patient's lungs requiring treatment. As shown in FIG. 4 (described further below), the 3D model may include, among other things, a model airway tree 402 corresponding to the actual airways of the patient's lungs, and show the various passages, branches, and bifurcations of the patient's actual airway tree. Additionally, the 3D model may include lesions 420, markers, blood vessels and vascular structures 404, lymph nodes and other lymphatic structures 410, organs, other physiological structures, and/or a 3D rendering of the pleural surfaces 406 and fissures 408 of the patient's lungs. Some or all of the aforementioned elements may be selectively displayed, such that the clinician may choose which elements should be displayed when viewing the 3D model. Further, as described below, one or more 3D renderings may be generated based on the ultrasound image data acquired by the ultrasound sensor 63 of the EBUS 62, and these 3D renderings may additionally be displayed in conjunction with or separate from the 3D model.

During a procedure, EM sensor 94, in conjunction with tracking system 70, enables tracking of EM sensor 94 (and thus distal tip 93 of EWC 96 or tools 62, 64) as EM sensor 94 is advanced through the patient's airways following the pathway planned during the planning phase of the EMN procedure. As an initial step of the procedure, the 3D model is registered with the patient's actual airways. One potential method of registration involves navigating LG 92 (or another tool including the EM sensor 94) into each lobe of the patient's lungs to at least the second bifurcation of the airways of that lobe. The position of LG 92 is tracked during this registration phase, and the 3D model is iteratively updated based on the tracked position of the locatable guide within the actual airways of the patient's lungs. This registration process is described in commonly-assigned U.S. Patent Appl. Publ. No. 2011/0085720, entitled "AUTOMATIC REGISTRATION TECHNIQUE," filed on May 14, 2010, by Barak et al., and U.S. Patent Appl. Publ. No. 2016/0000356, entitled "REAL-TIME AUTOMATIC REGISTRATION FEEDBACK", filed on July 2, 2015, by Brown et al., the entire contents of each of which are incorporated herein by reference. While the registration process focuses on aligning the patient's actual airways with the airways of the 3D model, registration also ensures that the position of vascular structures, lymphatic structures, pleural surfaces, and fissures of the lungs are accurately determined.

At various times during the procedure, the EBUS 62 may acquire ultrasound image data of various portions of the patient's chest, such as lesions, lymph nodes, and/or other structures. The computing device 80 may then generate the aforementioned 3D renderings of lesions, lymph nodes, and/or other structures based on the ultrasound image data. The computing device 80 may then register the 3D renderings to the 3D model based on the known position of the EBUS 62 while the ultrasound image data are obtained (based on the EM sensor 94 coupled to the EBUS 62).

The computing device 80 may then update and/or enhance the 3D model based on the ultrasound image data and/or the 3D renderings. The computing device 80 may further update and/or enhance the registration of the 3D model to the patient's body based on the registration of the 3D renderings to the 3D model. For example, the ultrasound image data may provide additional clarity and/or identify structures that are not visible in the radiographic image data and/or the 3D model, and the positions of such additional structures may be used to improve the registration of the 3D model to the patient's body. The computing device 80 may then generate a plan for obtaining biopsy samples from one or more of the lesions or lymph nodes of which 3D renderings were generated.

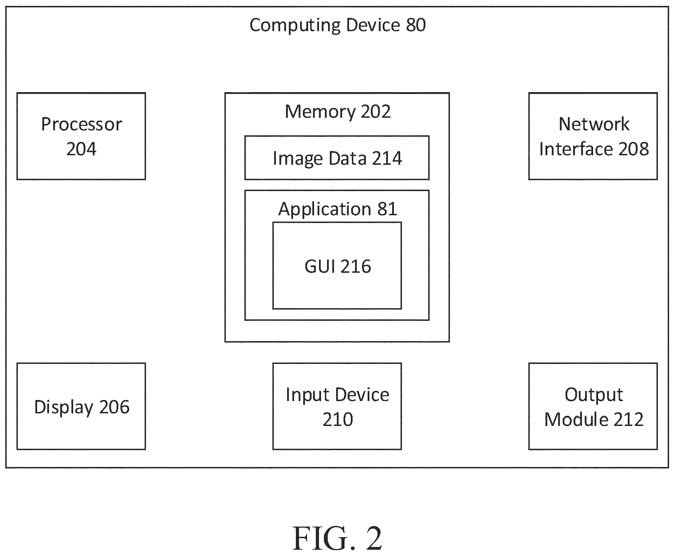

Turning now to FIG. 2, there is shown a simplified block diagram of computing device 80. Computing device 80 may include a memory 202, a processor 204, a display 206, a network interface 208, an input device 210, and/or an output module 212. Memory 202 may store the application 81 and/or image data 214. The application 81 may, when executed by the processor 204, cause the display 206 to present a graphical user interface (GUI) based on GUI instructions 216. The application 81 may also provide the interface between the tracked position of EM sensor 94 and the image and planning data developed in the pathway planning phase.

The memory 202 may include any non-transitory computer-readable storage media for storing data and/or software that is executable by the processor 204 and which controls the operation of the computing device 80. In an embodiment, the memory 202 may include one or more solid-state storage devices such as flash memory chips. Alternatively or in addition to the one or more solid-state storage devices, the memory 202 may include one or more mass storage devices connected to the processor 204 through a mass storage controller (not shown) and a communications bus (not shown). Although the description of computer-readable media contained herein refers to a solid-state storage, it should be appreciated by those skilled in the art that computer-readable storage media can be any available media that can be accessed by the processor 204. That is, computer readable storage media includes non-transitory, volatile and non-volatile, removable and non-removable media implemented in any method or technology for storage of information such as computer-readable instructions, data structures, program modules or other data. For example, computer-readable storage media includes RAM, ROM, EPROM, EEPROM, flash memory or other solid state memory technology, CD-ROM, DVD, Blu-Ray or other optical storage, magnetic cassettes, magnetic tape, magnetic disk storage or other magnetic storage devices, or any other medium which can be used to store the desired information and which can be accessed by the computing device 80.

The network interface 208 may be configured to connect to a network such as a local area network (LAN) consisting of a wired network and/or a wireless network, a wide area network (WAN), a wireless mobile network, a Bluetooth network, and/or the internet. The input device 210 may be any device by means of which a user may interact with the computing device 80, such as, for example, a mouse, keyboard, foot pedal, touch screen, and/or voice interface. The output module 212 may include any connectivity port or bus, such as, for example, parallel ports, serial ports, universal serial busses (USB), or any other similar connectivity port known to those skilled in the art.

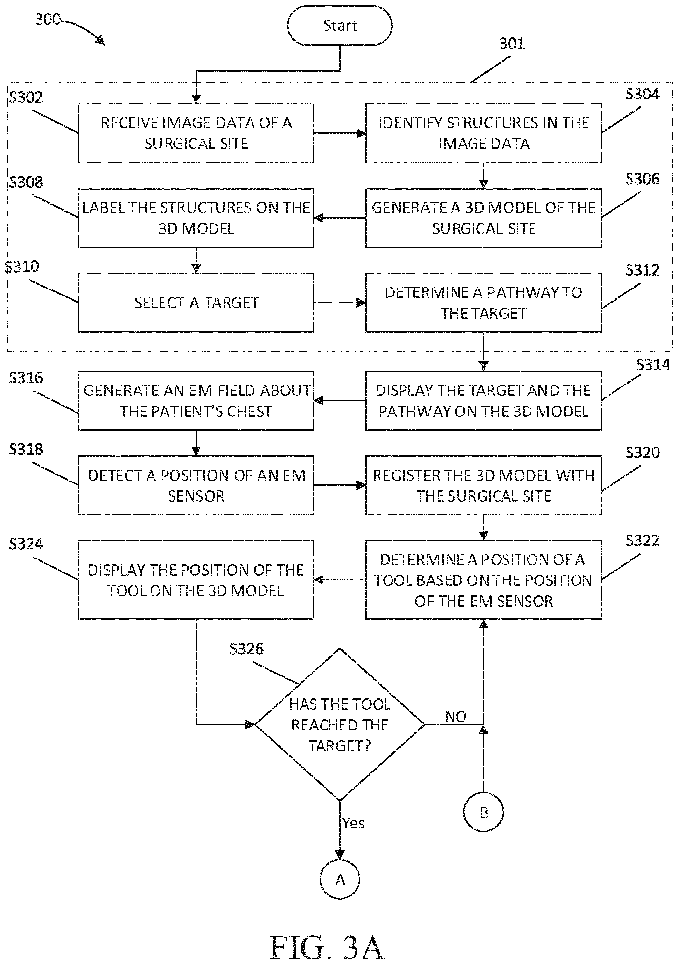

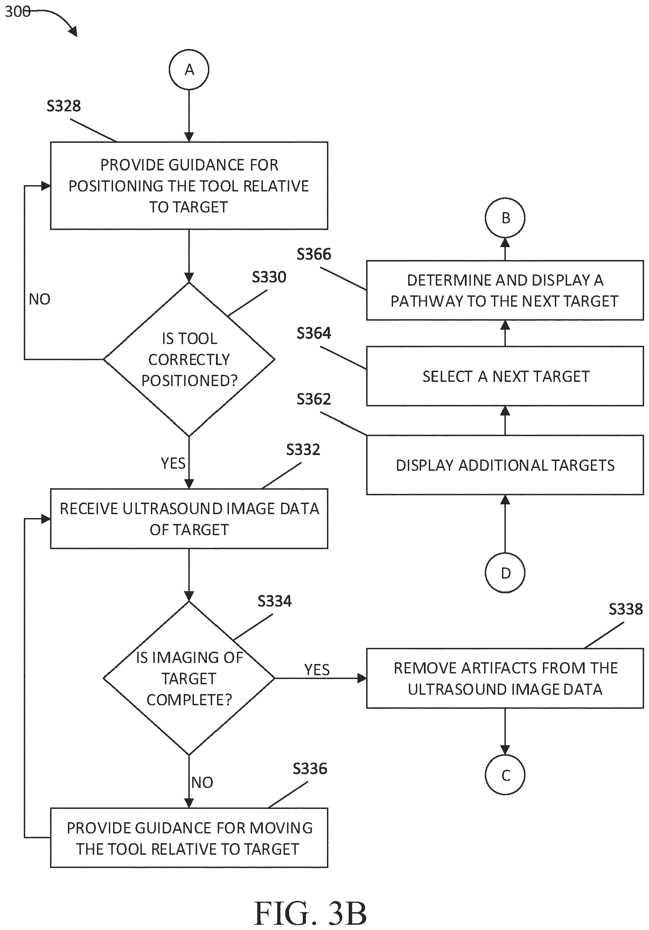

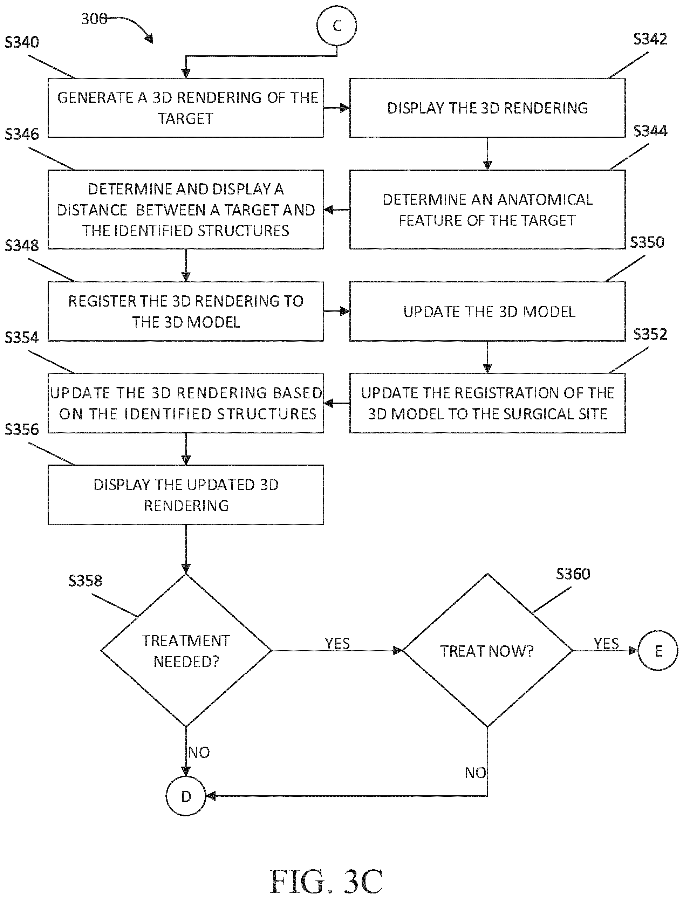

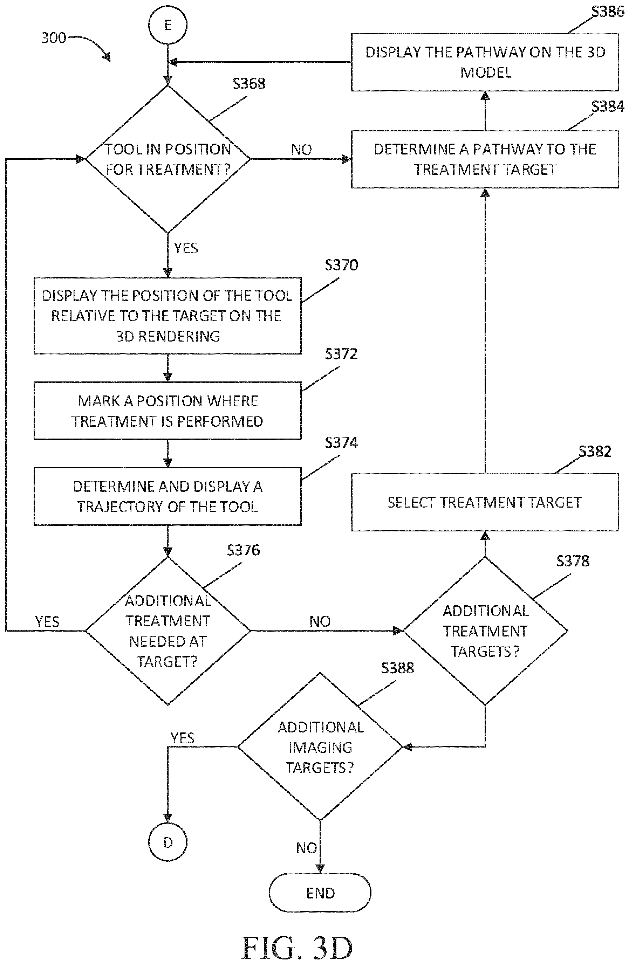

Turning now to FIGS. 3A, 3B, 3C, and 3D (referred to collectively as FIG. 3), there is shown a flowchart of an illustrative method 300 of performing diagnosis and/or treatment of an area of a patient's lungs, in accordance with an embodiment of the present disclosure. The method 300 includes various steps described in an ordered sequence. However, those skilled in the art will appreciate that one or more steps of the method 300 may be performed in a different order, repeated, and/or omitted without departing from the scope of the present disclosure. Further, the below description of the method 300 refers to various actions or tasks performed by the computing device 80, but those skilled in the art will appreciate that in some instances, the computing device 80 performs the actions or tasks via one or more software applications, such as the application 81, executing on the computing device 80.

The method 300 may begin with a planning phase 301including various steps that may be performed prior to a patient being placed on the table 40 for the diagnostic and/or treatment procedure. For example, the patient may undergo radiographic imaging and the radiographic image data processed by the computing device 80 prior to the patient coming in for the diagnostic and/or treatment procedure. In other embodiments, the steps of the planning phase 301 may be performed as part of a system configuration while the patient is already on the table 40, and thus the patient may remain in the same position after the radiographic imaging is performed.

Starting at step S302, the computing device 80 receives first image data a surgical site. As noted above, the surgical site includes at least a portion of the patient's body. For illustrative purposes, the description below will use the patient's lungs as the surgical site. In some embodiments, the first image data may include image data from multiple pre-operative scans. In other embodiments, only image data from a most recent scan may be used. The first image data may be received in, or converted to, a uniform data format, such as the digital imaging and communications in medicine (DICOM) standard. For example, the first image data may include image data from a CT scan, a CBCT scan, a MRI scan, a PET scan, an X-ray scan, etc.

Next, at step S304, the computing device 80 processes the first image data to identify one or more structures in the first image data. For example, the computing device 80 may identify the patient's lungs, and particularly, the bronchial network of the patient's airways in the first image data. The computing device 80 may further identify one or more lumens of the patient's vascular system, one or more lymph nodes and/or ducts of the patient's lymphatic system, other organs, markers, and/or one or more cysts or lesions or other aberrant structures in the first image data, as well as the pleural surfaces and/or fissures of the patient's lungs. The image processing may include automatic and/or user-assisted image analysis to identify the structures in the first image data. Various image processing methods may be used, including region growing techniques, as described in commonly-owned U.S. Patent Appl. Publ. No. 2016/0038248, entitled "TREATMENT PROCEDURE PLANNING SYSTEM AND METHOD", filed on August 10, 2015, by Bharadwaj et al., and commonly-owned U.S. Patent Appl. Publ. No. 2016/0005193, entitled "SYSTEM AND METHOD FOR SEGMENTATION OF LUNG", filed on June 30, 2015, by Markov et al., the entire contents of each of which are incorporated herein by reference.

Thereafter, at step S306, application 81 generates a three-dimensional (3D) model of the surgical site. The 3D model includes graphical representations of the surgical site, such as the patient's lungs, showing the locations of the lumens and structures of the bronchial, vascular, and lymphatic trees, as well as the pleural surfaces and fissures of the patient's lungs, markers, and/or lesions or other aberrant structures that may be present in the patient's lungs, as was identified in step S304. Next, at step S308, the computing device 80 labels one or more of the structures identified at step S304 in the model generated at step S306. In embodiments, the computing device 80 may label one or more lymph nodes based on a predetermined naming scheme or convention, such as based on the International Association for the Study of Lung Cancer's (IASLC) lymph node map. Similarly, the computing device 80 may label the various branches of the bronchial and/or vascular networks based on predetermined naming schemes or conventions.

Then, at step S310, a target is selected. The target may be selected from among the structures identified at step S304. The target may be automatically selected by the computing device 80, semi-automatically, and/or manually by the clinician providing input to the computing device 80, such as via input device 210. In embodiments, the computing device 80 may highlight (or in some other way display) one or more areas as potential lesions and/or tumors detected via image analysis of the first image data received at step S302 for review by the clinician. The clinician may then confirm whether the highlighted areas are lesions and provide input to the computing device 80 to mark the confirmed lesions as targets in the 3D model. The clinician may also select one or more lesions and/or targets by viewing the first image data and/or the 3D model. For example, by using input device 210 and display 206 of the computing device 80, the clinician may view the first image data and/or 3D model and may identify and select one or more lesions and/or targets. The clinician may also select and/or mark various areas of the first image data to identify those areas as areas that may require diagnosis and/or treatment. The computing device may then identify and mark one or more areas in the 3D model that correspond to the areas marked by the clinician. In some embodiments, a plurality of targets are selected at step S310 and ordered in a list. For example, the computing device 80 may select a plurality of lymph nodes identified at step S304 as the targets. Additional details regarding the identification of structures and selection of targets are described in commonly-assigned U.S. Provisional Patent Appl. No. 62/624,905, which is incorporated above.

After a target is selected, the computing device 80, at step S312, determines a pathway to the target. In embodiments, the computing device 80 determines a pathway via a luminal network, such as the patient's airways, from the target to the patient's trachea. In embodiments where the target is situated in the parenchyma surrounding the airways, at least a portion of the pathway may be outside of the airways to connect the target with the remaining portion of the pathway inside the airways. In embodiments where multiple targets are selected, a plurality of pathways may be determined to visit each target. Additionally, the computing device 80 may automatically, or with input from the clinician, generate a diagnosis and/or treatment plan based on the identified structures, the selected targets, and/or the pathway, as described further in U.S. Patent Appl. Publ. No. 2016/0038248, noted above. As will be appreciated by those skilled in the art, consistent with the current iLogic™ planning system described in U.S. Patent Appl. Publ. No. 2016/0038248, this diagnosis and/or treatment plan generation may also occur prior to the generation of the 3D model by simply viewing the first image data, without departing from the scope of the present disclosure.

Following step S312, a navigation phase, as described above, may commence. Those of skill in the art will recognize that the planning phase may occur as a separate action from the navigation phase (e.g., at some date or time prior to the actual procedure). The navigation phase may include the endobronchial navigation of the LG 92 and/or the EBUS 62 of the system 100 to the target selected at step S310 via the pathway determined at step S312. As an initial step of the navigation phase a navigation plan is selected and loaded for display of the target and the pathway on the 3D model at step S314. In embodiments, the computing device 80 may cause a display device, such as display 206, to display the 3D model with the target and the pathway indicated thereon.

Thereafter, at step S316, the EM field generator 76 of the EM tracking system 70 generates an EM field about the patient's body, and in particular, about the patient's chest. The EM tracking system 70 then, at step S318, detects a position of the EM sensor 94 in the EM field. The EM tracking system 70 then provides EM tracking data regarding the detected position of the EM sensor 94 to the computing device 80.

The 3D model may then, at step S320, be registered with the patient's body, as described above. Alternatively, in embodiments where the first image data are obtained at the start of the diagnostic and/or treatment procedure after the patient is already positioned on the table 40, the 3D model generated at step S306 may not need to be registered with the patient's body because the first image data received at step S302 show the patient in the current position of the patient's body on the table 40. In such embodiments, the 3D model is merely aligned with the patient's body, such as via the reference sensors 74 or other markers placed on the patient's body prior to obtaining the first image data.

In either embodiment, after the 3D model has been registered or aligned with the patient's body, the computing device 80, at step S322, determines a position of the LG 92 and/or EBUS 62 based on the EM tracking data received from the EM tracking system at step S318. The computing device 80 then, at step S324, displays the tracked position of EM sensor 94 on the 3D model, thereby providing an indication of the position of the LG 92 and/or EBUS 62 inside the patient's airways. In embodiments, the computing device 80 causes the display 206 to display the tracked position of the LG 92 and/or EBUS 62 on the 3D model.

Thereafter, at step S326, the computing device 80 determines whether the LG 92 and/or EBUS 62 have reached the target. For example, the computing device 80 may determine whether the EM tracking data received from the EM tracking system indicates that the EM sensor 94 is proximate the position of the target selected at step S310. If the computing device 80 determines that the LG 92 and/or EBUS 62 have not reached the target ("NO" at step S326), processing returns to step S322. Alternatively, if the computing device 80 determines that the LG 92 and/or EBUS 62 have reached the target ("YES" at step S326), processing proceeds to step S328.

At step S328, the computing device 80 provides guidance for positioning the LG 92 and/or EBUS 62 relative to the target. For example, the computing device 80 may cause the display 206 to display visual guidance for positing the LG 92 and/or EBUS 62 relative to the target. The guidance may include visual and/or audible instructions for positioning the LG 92 and/or EBUS 62. The computing device 80 then, at step S330, determines whether the LG 92 and/or EBUS 62 are correctly positioned relative to the target. For example, the computing device 80 may determine whether the EM tracking data received from the EM tracking system 70 indicates that the EM sensor 94 is at a correct position and/or orientation (referred to hereinafter as a "pose") relative to the target. If the computing device 80 determines that the LG 92 and/or EBUS 62 is not correctly positioned relative to the target ("NO" at step S330), processing returns to step S328. Alternatively, if the computing device 80 determines that the LG 92 and/or EBUS 62 are correctly positioned relative to the target ("YES" at step S330), processing proceeds to step S332. After the computing device 80 determines that the LG 92 is correctly positioned relative to the target, the computing device 80 may provide guidance for removing the LG 92 from the EWC 96 and inserting the EBUS 62 into the EWC 96. Alternatively, if the EBUS 62 was used to navigate to the target, no tool exchange is necessary at this stage.

At step S332, the computing device 80 receives ultrasound image data of the target. The ultrasound image data may be captured by the EBUS 62 and may be provided to the computing device 80 via an ultrasound workstation and/or a direct connection between the EBUS 62 and the computing device 80. After receiving the ultrasound image data, the computing device 80, at step S334, determines whether ultrasound imaging of the target is complete. For example, the computing device 80 may receive input from the clinician indicating that the ultrasound imaging is complete. Additionally or alternatively, the computing device 80 may determine whether the entire target can be identified in the ultrasound image data in order to determine whether the ultrasound imaging of the target is complete. If the computing device 80 determines that the ultrasound imaging of the target is not complete ("NO" at step S334), processing proceeds to step S336, where the computing device 80 provides guidance for moving the EBUS 62 relative to the target. For example, the computing device 80 may cause the display 206 to display visual guidance for moving the EBUS 62 relative to the target. Thereafter, processing returns to step S332.

Alternatively, if the computing device 80 determines that the ultrasound imaging of the target is complete ("YES" at step S334), processing proceeds to step S338. At step S338, the computing device 80 processes the ultrasound image data received at step S332 to remove artifacts from the ultrasound image data. The computing device 80 may further process the ultrasound image data to identify the target in the ultrasound image data. Thereafter, at step S340 the computing device 80 generates a 3D rendering based on the target based on the ultrasound image data. For example, the computing device 80 may use various image processing algorithms, including segmentation and region growing algorithms, to identify the target in the ultrasound image data and stitch together various portions of the ultrasound image data to generate a 3D rendering of the target. The computing device 80 may display the 3D rendering of the target at step S342. For example, the computing device 80 may cause the display 206 to display the 3D rendering of the target.

The computing device 80 may further determine, at step S344, one or more anatomical features of the target based on the ultrasound image data and/or the 3D rendering of the target. The anatomical features may include a size, a shape, a margin, an echogenicity, a central hilar structure, and/or a coagulation necrosis characteristic of the target. Additionally, the computing device 80 may determine, at step S346, a distance, direction, and/or interaction between the target and one or more of the structures identified at step S304. The computing device 80 may then cause the display 206 to display an indication of the distance, direction, and/or interaction between the target and the structures identified at step S304.

Thereafter, at step S348, the computing device 80 registers the 3D rendering of the target with the 3D model. In embodiments, the computing device 80 registers the 3D rendering of the target with the 3D model based on the position of the EBUS 62 when the ultrasound image data were obtained, as determined based on the position of the EM sensor 94. The computing device 80 may then, at step S350, update and/or enhance the portion of the 3D model corresponding to the area for which ultrasound image data were received. For example, the computing device 80 may process the ultrasound image data to identify structures in the ultrasound image data that are imaged with greater detail or clarity than the first image data received at step S302. The computing device 80 may then update and/or enhance the 3D model based on such structures. Additionally, the computing device 80 may, at step S352, update the registration of the 3D model to the patient's body based on the ultrasound image data received at step S332 and/or the structures identified therein. The computing device 80 may further, at step S354, update and/or enhance the 3D rendering of the target based on the 3D model, the first image data, and/or the structures identified at step S304. The computing device 80 may then, at step S356, display the updated 3D rendering of the target. For example, the computing device 80 may cause the display 206 to display the updated 3D rendering in conjunction with or separate from the 3D model.

Thereafter, at step S358, the computing device 80 determines whether treatment of the target is needed. In embodiments, the computing device 80 determines whether treatment of the target is needed based on input provided by the clinician, such as via input device 210. In other embodiments, the computing device 80 may also determine whether treatment of the target is needed based on the predetermined diagnostic and/or treatment plan, and/or based on the anatomical feature of the target determined at step S344. If the computing device 80 determines that treatment of the target is needed ("YES" at block 358), processing proceeds to block S360.

At block S360, the computing device 80 determines whether the treatment of the target is to be performed now. In embodiments, the computing device 80 determines whether the treatment of the target is to be performed now based on input provided by the clinician, such as via input device 210. In other embodiments, the computing device 80 determines whether the treatment is to be performed now based on the predetermined diagnostic and/or treatment plan and/or based on the position of the EBUS 62 relative to the target. For example, the EBUS 62 may be positioned in a lumen or pose that is favorable for capturing the ultrasound image data, but may not be favorable for performing treatment of the target. Other considerations that may affect the determination of whether the treatment is the be performed now includes requirements for tool exchanges, time constraints, and/or additional navigation or positioning required. If the computing device 80 determines that the treatment is to be performed now ("YES" at step S360), processing proceeds to step S368. Alternatively, if the computing device 80 determines that the treatment is not needed ("NO" at step S358), or that the treatment is not to be performed now ("NO" at step S360), processing proceeds to step S362, where the computing device 80 displays the additional targets. In embodiments, the computing device 80 may cause the display 206 to a view of the 3D model showing the additional targets. For example, the additional targets may be highlighted and/or displayed with a different characteristic, such as a different color, than targets that have already been imaged. In some embodiments, one or more regions of the 3D model may be displayed with a different characteristic, such as a different color, to indicate that there are additional targets to be imaged in those regions of the 3D model. Thereafter, at step S364, the computing device 80 selects (automatically, semi-automatically, or manually) a next target. The next target may be selected based on the predetermined diagnosis and/or treatment plan, based on its proximity to the previous target, based on its accessibility from the current position of the EBUS 62, etc. The computing device 80 then, at step S366, determines a pathway from the current position of the EBUS 62 to the next target. The computing device 80 may further cause the display 206 to display the pathway to the next target on the 3D model. Thereafter, processing returns to step S322.

At step S368, the computing device 80 determines whether the treatment tool is in the correct pose relative to the target to perform the treatment. For example, the computing device 80 may determine whether the EM tracking data received from the EM tracking system 70 indicates that the EM sensor 94 is in the correct pose relative to the target to perform the treatment. In embodiments where a biopsy tool separate from the EBUS 62 is used, or where a different type of treatment tool, such as the ablation tool 64, is needed, a tool exchange may be required. If the computing device 80 determines that the treatment tool is not in the correct pose relative to the target ("NO" at step S368), processing proceeds to step S384, where the computing device 80 determines a pathway to the target and/or guidance for manipulating the treatment tool into the correct pose relative to the target. Thereafter, at step S386, the computing device 80 displays the pathway and/or guidance determined at step S384, such as via the display 206. Then, processing returns to step S368.

Alternatively, if the computing device 80 determines that the treatment tool is correctly positioned relative to the target to perform the treatment ("YES" at step S368), processing proceeds to step S370.

The computing device 80 then tracks the position of the treatment tool, based on EM tracking data received from the EM tracking system 70, as the treatment tool is navigated to the biopsy target, and displays the current position of the treatment tool on the 3D model.

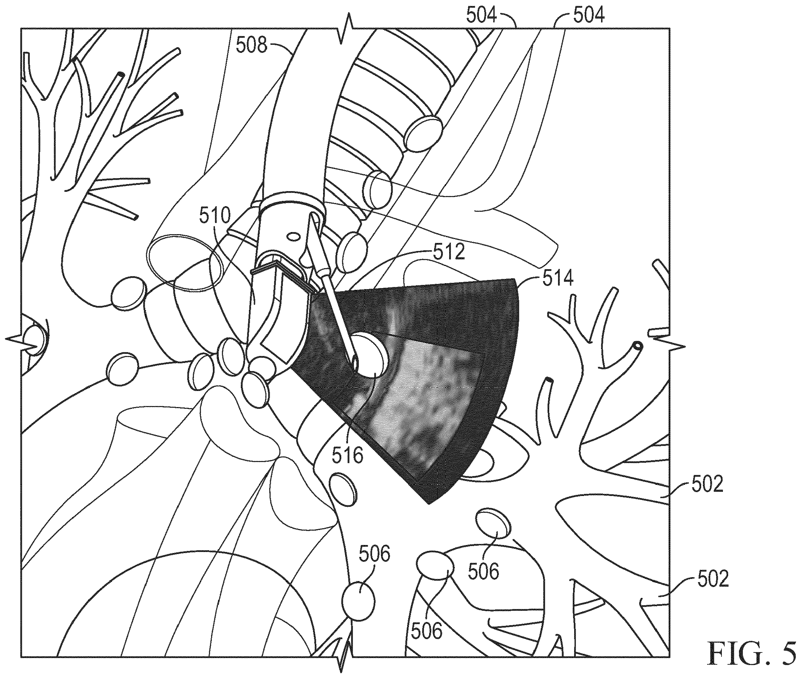

At step S370, the computing device 80 displays a view of the 3D rendering showing the position of the treatment tool relative to the target. In embodiments, the computing device 80 may cause the display 206 to display a view of the 3D rendering showing the pose of the treatment tool relative to the target and any interaction between the treatment tool and the target. For example, as shown in FIG. 5, a view of the 3D model may be displayed showing the pose of an EBUS 508 relative to a target 516, and interaction between a biopsy needle 512 and the target 516.

Once the treatment has been performed, the computing device 80, at step S372, marks the position where the treatment was performed on the 3D model and/or the 3D rendering of the target. For example, the computing device 80 may place a digital marker on the 3D model and/or the 3D rendering of the target indicating the exact pose of the EBUS 62 and/or the biopsy tool when the biopsy was performed. Alternatively, if the treatment is an ablation procedure, the computing device may mark the pose of the ablation tool 64 when the ablation procedure was performed on the 3D model. The marker may later be updated with diagnosis information once the tissue sample has been analyzed or once additional diagnostic or treatment data is available.

Next, at step S374, the computing device 80 determines a trajectory of the treatment tool. For example, the computing device 80 may determine, based on the EM tracking data received from the EM tracking system 70, the direction in which the EBUS 62 and/or the biopsy tool will move if the EBUS 62 is further extended in the current trajectory. The computing device 80 may further determine based on the trajectory whether additional treatment locations of the current target are reachable along the current trajectory of the treatment tool. The computing device 80 may then display the trajectory of the treatment tool on the 3D model and/or the 3D rendering of the target.

Thereafter, at step S376, the computing device 80 determines (automatically, semi-automatically, or manually) whether additional treatment is needed at the current target. For example, the computing device 80 may determine whether another biopsy sample is needed at the current target or whether additional ablation is needed. If the computing device 80 determines that additional treatment is needed at the current target ("YES" at step S376), processing returns to step S368. Alternatively, if the computing device 80 determines that additional treatment is not needed at the current target ("NO" at step S376), processing proceeds to step S378.

At step S378, the computing device 80 determines whether there are additional targets requiring treatment. For example, the computing device 80 may determine whether there are additional targets requiring treatment based on input provided by the clinician, and/or based on the predetermined diagnostic and/or treatment plan. If the computing device determines that there are additional targets requiring treatment ("YES" at step S378), processing proceeds to step S382, where the computing device 80 selects the next treatment target, whereafter processing proceeds to step S284. Alternatively, if the computing device 80 determines that there are no additional treatment targets remaining ("NO" at step S378), processing proceeds to step S388.

At step S388, the computing device 80 determines whether there are additional targets remaining that require imaging. In embodiments, the computing device 80 may determine whether there are additional targets remaining that require imaging based on input provided by the clinician, and/or based on the predetermined diagnostic and/or treatment plan. If the computing device determines that there are additional targets remaining that require imaging ("YES" at step S388), processing returns to step S362. Alternatively, if the computing device determines that there are no additional targets remaining that require imaging,("NO" at step S388), the method 300 ends.

FIG. 4 shows an illustrative graphical user interface (GUI) including a 3D model of a patient's chest showing portions of the bronchial and vascular trees, as well as various lymph nodes and the pleura and fissures of the patient's lungs, as described above. The 3D model includes a bronchial tree 402 showing the trachea and the various bifurcations of the airways, and the pleural surfaces 406 and fissures 408 of the patient's lungs. The 3D model further includes vascular structures 404, such as major arteries and veins, as well as lymph nodes 410, and a selected target location 420.

FIG. 5 shows another illustrative GUI that may be displayed during various steps of the method 300. As noted above, FIG. 5 shows a view of the 3D model including portions of the patient's bronchial tree 502, vascular tree 504, and lymph nodes 506. FIG. 5 also shows a representation of the EBUS 508 including an ultrasound sensor 510 and a biopsy needle 512. Ultrasound images 514 captured by the ultrasound sensor are shown overlaid onto the 3D model, with a 3D rendering of the target 516 displayed thereon.

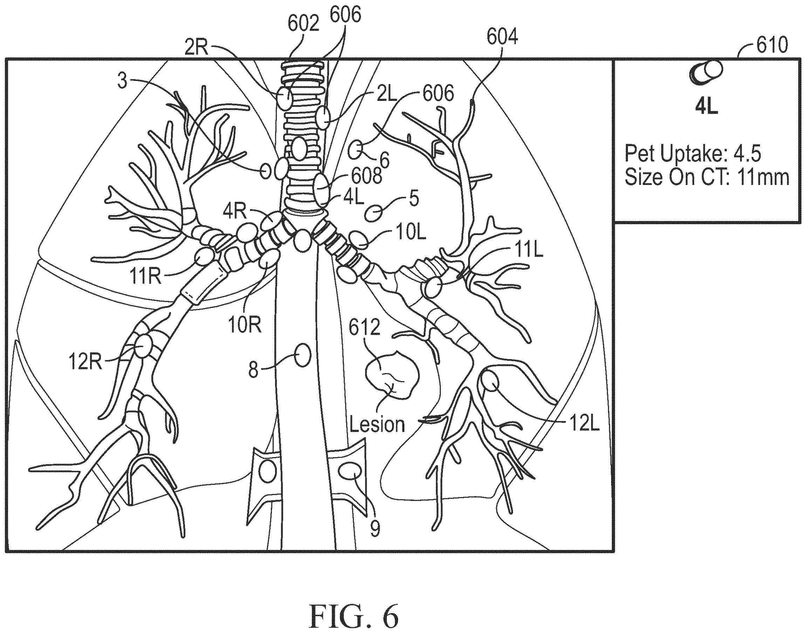

FIG. 6 shows yet another illustrative GUI that may be displayed during various steps of the method 300. Similar to FIGS. 4 and 5, FIG. 6 also shows a view of the 3D model including the patient's trachea 602 and airway tree 604, along with the positions of a plurality of lymph nodes 606 displayed thereon. In embodiments, the lymph nodes 606 are overlaid onto the 3D model. One of the lymph nodes 608 may be selected and may be displayed with a different visual characteristic (such as a different color). Additional details, such as anatomical features or characteristics, regarding the selected lymph node 608 may be displayed in a view 610. The additional details may include a PET uptake, a size, etc. Additional structures, such as one or more lesions 612 may also be displayed on the view of the 3D model.

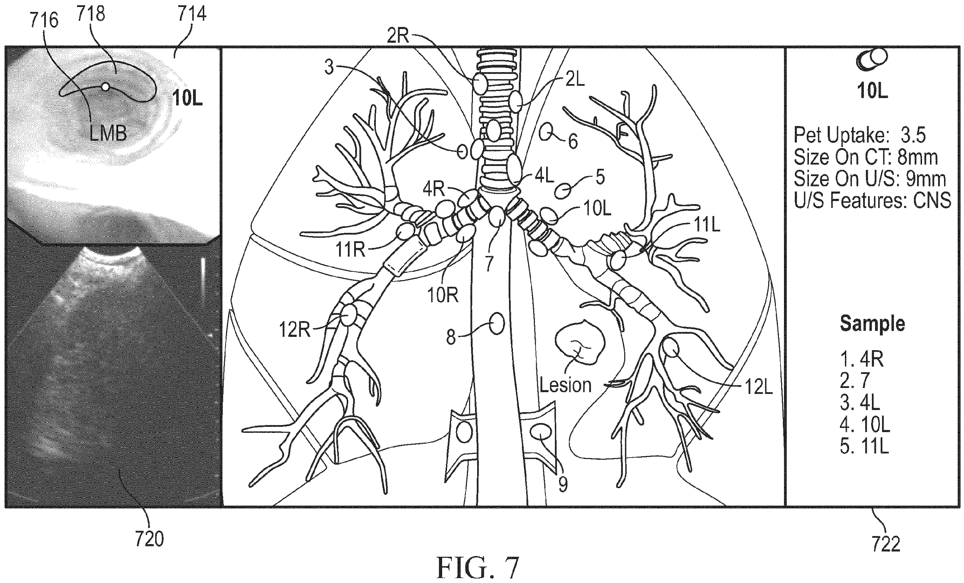

FIG. 7 shows another view of the GUI of FIG. 6 showing additional features and/or details that may be displayed during various steps of the method 300. Features of FIG. 7 that were described above with reference to FIG. 6 will not be described again for purpose of brevity. In addition to the features described in FIG. 6, FIG. 7 further includes a bronchoscopic view 714 showing live video images received from an imaging device included in the bronchoscope 50. The bronchoscopic view 714 may be overlaid with a label 716 indicating the portion of the bronchial tree that is displayed in the bronchoscopic view 714, as well as an overlay 718 indicating the position of various structures, such as the selected lymph node 608, relative to the shown airway tree. FIG. 7 further shows a view of the ultrasound image data 720 captured by the EBUS 62. Additionally, FIG. 7 may show a list 722 of lymph nodes for which treatment is required.

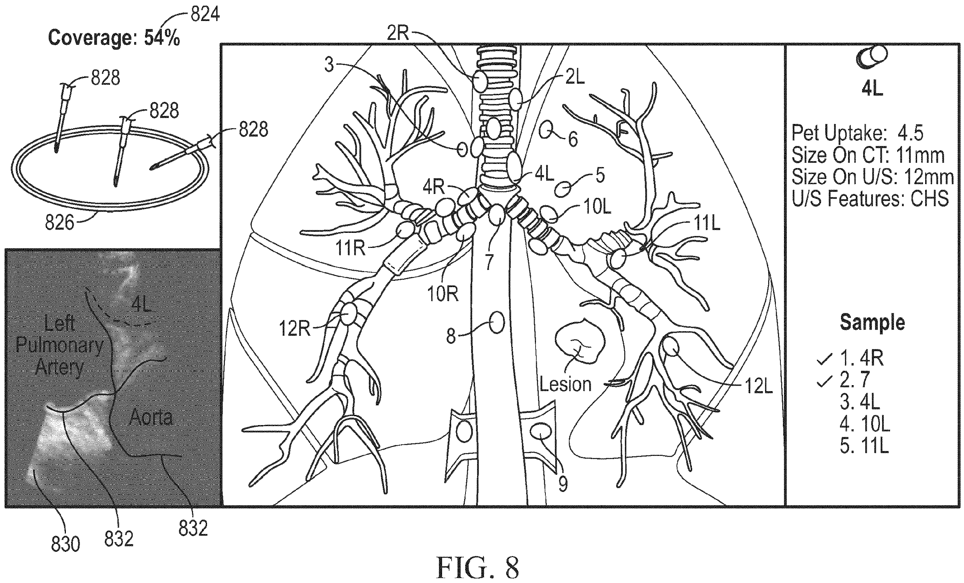

FIG. 8 shows yet another view of the GUI of FIG. 6 and 7 showing additional features and/or details that may be displayed during various steps of the method 300. Features of FIG. 8 that were described above with reference to FIGS. 6 and 7 will not be described again for purpose of brevity. In addition to the features described in FIGS. 6 and 7, FIG. 8 shows a view 824 of the 3D rendering 826 of a selected lymph node 608. The view 824 of the 3D rendering 826 may show markers 828 indicating positions where previous treatments were performed. For example, the view 824 of the 3D rendering 826 may show markers representing the pose of a biopsy needle when a tissue sample was obtained. FIG. 8 further shows a view 830 of the ultrasound image data captured by the EBUS 62, where the ultrasound image data is augmented with indicators 832 representing the positions of identified structures in the ultrasound image data.

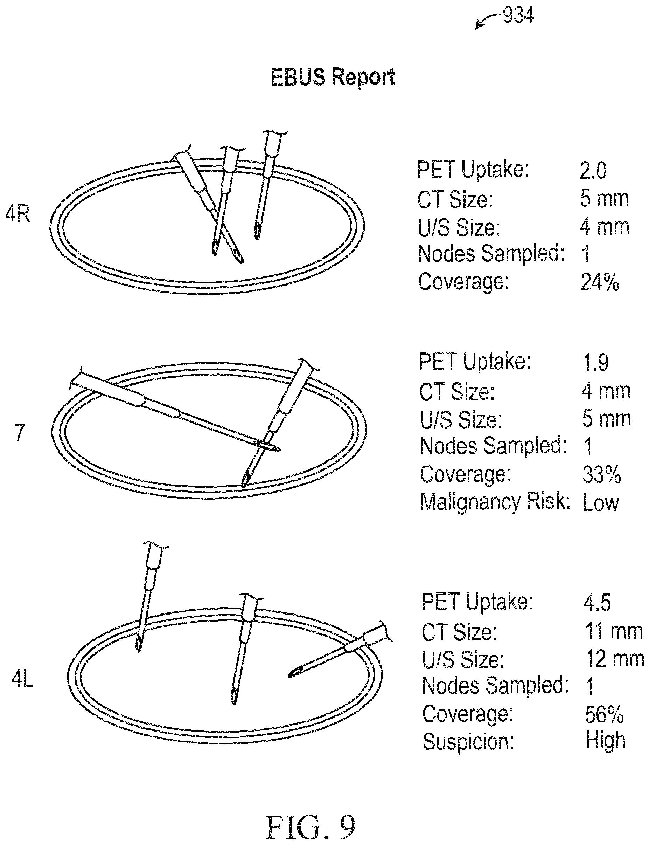

FIG. 9 shows a view of a GUI that may be shown during or after the various steps of the method 300. FIG. 9 includes a report 934 providing details of one or more selected lymph nodes 608, including the 3D renderings 826 of the selected lymph nodes 608, as well as the additional details 610 regarding the selected lymph nodes, including a PET uptake, a size of the selected lymph node 608 on a CT image, a size of the selected lymph node on an ultrasound image, an indicating of whether and/or how many times the selected lymph node 608 has been treated or sampled, coverage of the treatment, a malignancy risk of the selected lymph node 608, and/or a suspicion of being diseased and/or requiring treatment.

Detailed embodiments of devices, systems incorporating such devices, and methods using the same as described herein. However, these detailed embodiments are merely examples of the disclosure, which may be embodied in various forms. Therefore, specific structural and functional details disclosed herein are not to be interpreted as limiting, but merely as a basis for the claims and as a representative basis for allowing one skilled in the art to variously employ the present disclosure in appropriately detailed structure. While the preceding embodiments are described in terms of bronchoscopy of a patient's airways, those skilled in the art will realize that the same or similar devices, systems, and methods may be used in other lumen networks, such as, for example, the vascular, lymphatic, and/or gastrointestinal networks as well.

While several embodiments of the disclosure have been shown in the drawings, it is not intended that the disclosure be limited thereto, as it is intended that the disclosure be as broad in scope as the art will allow and that the specification be read likewise. Therefore, the above description should not be construed as limiting, but merely as exemplifications of particular embodiments. Those skilled in the art will envision other modifications within the scope and spirit of the claims appended hereto.

The invention may be described by reference to the following numbered paragraphs:-
1. A surgical system comprising:
   an electromagnetic (EM) tracking system including a EM field generator configured to generate an EM field about a surgical site;
   a surgical tool including an ultrasound sensor and an EM sensor;
   a display device; and
   a computing device including a processor and a memory storing instructions which, when executed by the processor, cause the computing device to:
      receive first image data of the surgical site;
      identify a structure in the first image data;
      identify a target in the first image data;
      generate a three-dimensional (3D) model of the surgical site based on the first image data and the identified structure and target;
      determine a pathway to the target;
      cause the display device to display the pathway to the target;
      determine a position of the surgical tool within the surgical site based on tracking data received from the EM tracking system, the tracking data indicating a position of the EM sensor within the EM field;
      register the 3D model to the surgical site;
      receive second image data from the ultrasound sensor;
      generate a 3D volume rendering of the target; and
      cause the display device to display the 3D volume rendering.
2. The system according to paragraph 1, wherein the surgical tool further includes an expandable balloon.
3. The system according to paragraph 1, wherein the instructions, when executed by the processor, further cause the computing device to cause the display device to display guidance for positioning the surgical tool relative to the target.
4. The system according to paragraph 1, wherein the instructions, when executed by the processor, further cause the computing device to cause the display device to display guidance for moving the surgical tool relative to the target while the ultrasound sensor captures the second image data.
5. The system according to paragraph 1, wherein the instructions, when executed by the processor, further cause the computing device to cause the display device to display a view of the 3D model showing regions of the surgical site for which second image data has been received with a first characteristic, and regions of the surgical site for which second image data has not been received with a second characteristic.
6. The system according to paragraph 1, wherein the instructions, when executed by the processor, further cause the computing device to cause the display device to display a view of the first image data showing regions of the surgical site for which second image data has been received with a first characteristic, and regions of the surgical site for which second image data has not been received with a second characteristic.
7. The system according to paragraph 1, wherein the instructions, when executed by the processor, further cause the computing device to process the second image data to remove artifacts from the second image data
8. The system according to paragraph 1, wherein the 3D volume rendering is generated based on at least a portion of the second image data and the determined position of the surgical tool.
9. The system according to paragraph 1, wherein the instructions, when executed by the processor, further cause the computing device to register the 3D volume rendering to the 3D model.
10. The system according to paragraph 1, wherein the instructions, when executed by the processor, further cause the computing device to register the 3D volume rendering to the first image data.
11. The system according to paragraph 1, wherein the instructions, when executed by the processor, further cause the computing device to update the 3D volume rendering based on the identified structure.
12. The system according to paragraph 1, wherein the structure includes one or more of a bronchial tree, a vascular tree, a lymphatic tree, a lesion, a cyst, an esophagus, a pleural surface, a lymph node, an organ, and a marker.
13. The system according to paragraph 1, wherein the instructions, when executed by the processor, further cause the computing device to cause the display device to display the updated 3D volume rendering.
14. The system according to paragraph 1, wherein the instructions, when executed by the processor, further cause the computing device to cause the display device to display an individual slice image of the 3D volume rendering.
15. The system according to paragraph 1, wherein the instructions, when executed by the processor, further cause the computing device to update the registration of the 3D model to the surgical site based on the registration of the 3D volume rendering to the 3D model.
16. The system according to paragraph 1, wherein the instructions, when executed by the processor, further cause the computing device to update the 3D model based on the second image data.
17. A surgical system comprising:
   an electromagnetic (EM) tracking system including a EM field generator configured to generate an EM field about a surgical site;
   a surgical tool including an ultrasound sensor and an EM sensor; and
   a computing device including a processor and a memory storing instructions which, when executed by the processor, cause the computing device to:
      receive first image data of the surgical site;
      generate a three-dimensional (3D) model of the surgical site based on an identified target in the image data;
      determine a pathway to the target;
      determine a position of the surgical tool within the surgical site based on a determined position of the EM sensor within the EM field;
      receive second image data from the ultrasound sensor; and
      generate a 3D volume rendering of the target based on the second image data.
18. The system according to paragraph 17, wherein the instructions, when executed by the processor, further cause the computing device to register the 3D volume rendering of the target to the 3D model of the surgical site.
19. A method for generating visual guidance for a surgical procedure, comprising:
   receiving first image data of a surgical site;
   generating a three-dimensional (3D) model of the surgical site;
   determining a pathway to a target identified in the first image data;
   displaying the pathway to the target on a display;
   determining a position of an electromagnetic (EM) sensor within an EM field generated about the surgical site;
   receiving second image data of the target from an ultrasound sensor; and
   generating a 3D volume rendering of the target based on the second image data.
20. The method according to paragraph 19, further comprising registering the 3D volume rendering of the target to the 3D model of the surgical site.

## Claims

1. A surgical system comprising:
an electromagnetic (EM) tracking system including a EM field generator configured to generate an EM field about a surgical site;
a surgical tool including an ultrasound sensor and an EM sensor;
a display device; and
a computing device including a processor and a memory storing instructions which, when executed by the processor, cause the computing device to:
receive first image data of the surgical site;
identify a structure in the first image data;
identify a target in the first image data;
generate a three-dimensional (3D) model of the surgical site based on the first image data and the identified structure and target;
determine a pathway to the target;
cause the display device to display the pathway to the target;
determine a position of the surgical tool within the surgical site based on tracking data received from the EM tracking system, the tracking data indicating a position of the EM sensor within the EM field;
register the 3D model to the surgical site;
receive second image data from the ultrasound sensor;
generate a 3D volume rendering of the target; and
cause the display device to display the 3D volume rendering.

2. The system according to claim 1, wherein the surgical tool further includes an expandable balloon.

3. The system according to claim 1, wherein the instructions, when executed by the processor, further cause the computing device to cause the display device to display guidance for positioning the surgical tool relative to the target; preferably wherein the instructions, when executed by the processor, further cause the computing device to cause the display device to display guidance for moving the surgical tool relative to the target while the ultrasound sensor captures the second image data.

4. The system according to any preceding claim, wherein the instructions, when executed by the processor, further cause the computing device to cause the display device to display a view of the 3D model showing regions of the surgical site for which second image data has been received with a first characteristic, and regions of the surgical site for which second image data has not been received with a second characteristic; and/or wherein the instructions, when executed by the processor, further cause the computing device to cause the display device to display a view of the first image data showing regions of the surgical site for which second image data has been received with a first characteristic, and regions of the surgical site for which second image data has not been received with a second characteristic.

5. The system according to any preceding claim, wherein the instructions, when executed by the processor, further cause the computing device to process the second image data to remove artifacts from the second image data; and/or wherein the 3D volume rendering is generated based on at least a portion of the second image data and the determined position of the surgical tool.

6. The system according to any preceding claim, wherein the instructions, when executed by the processor, further cause the computing device to register the 3D volume rendering to the 3D model; and/or wherein the instructions, when executed by the processor, further cause the computing device to register the 3D volume rendering to the first image data.

7. The system according to any preceding claim, wherein the instructions, when executed by the processor, further cause the computing device to update the 3D volume rendering based on the identified structure.

8. The system according to any preceding claim, wherein the structure includes one or more of a bronchial tree, a vascular tree, a lymphatic tree, a lesion, a cyst, an esophagus, a pleural surface, a lymph node, an organ, and a marker.

9. The system according to any preceding claim, wherein the instructions, when executed by the processor, further cause the computing device to cause the display device to display the updated 3D volume rendering.

10. The system according to any preceding claim, wherein the instructions, when executed by the processor, further cause the computing device to cause the display device to display an individual slice image of the 3D volume rendering.

11. The system according to any preceding claim, wherein the instructions, when executed by the processor, further cause the computing device to update the registration of the 3D model to the surgical site based on the registration of the 3D volume rendering to the 3D model.

12. The system according to any preceding claim, wherein the instructions, when executed by the processor, further cause the computing device to update the 3D model based on the second image data.

13. A surgical system comprising:
an electromagnetic (EM) tracking system including a EM field generator configured to generate an EM field about a surgical site;
a surgical tool including an ultrasound sensor and an EM sensor; and
a computing device including a processor and a memory storing instructions which, when executed by the processor, cause the computing device to:
receive first image data of the surgical site;
generate a three-dimensional (3D) model of the surgical site based on an identified target in the image data;
determine a pathway to the target;
determine a position of the surgical tool within the surgical site based on a determined position of the EM sensor within the EM field;
receive second image data from the ultrasound sensor; and
generate a 3D volume rendering of the target based on the second image data.

14. The system according to claim 13, wherein the instructions, when executed by the processor, further cause the computing device to register the 3D volume rendering of the target to the 3D model of the surgical site.

15. A method for generating visual guidance for a surgical procedure, comprising:
receiving first image data of a surgical site;
generating a three-dimensional (3D) model of the surgical site;
determining a pathway to a target identified in the first image data;
displaying the pathway to the target on a display;
determining a position of an electromagnetic (EM) sensor within an EM field generated about the surgical site;
receiving second image data of the target from an ultrasound sensor; and
generating a 3D volume rendering of the target based on the second image data;
preferably further comprising registering the 3D volume rendering of the target to the 3D model of the surgical site.
